# EUROPÄISCHE PATENTSCHRIFT

(11) **EP 3 337 339 B1**
(45) Veröffentlichungstag und Bekanntmachung des Hinweises auf die Patenterteilung: **11.03.2020**
(21) Anmeldenummer: 16757004.3
(22) Anmeldetag: 19.08.2016
(51) Int. Cl.: A23L 2/52, B01D 11/00, A61K 36/63, A23L 33/105

(54) **OLIVENBLATTEXTRAKT**
OLIVE LEAF EXTRACT
EXTRAIT DE FEUILLE D'OLIVIER

(30) Priorität: 21.08.2015 EP 15182024
(43) Veröffentlichungstag der Anmeldung: 27.06.2018
(73) Patentinhaber: Döhler GmbH, 64295 Darmstadt (DE)
(72) Erfinder: SARAFEDDINOV, Alla, 64646 Heppenheim (DE); ZOTZEL, Jens, 64291 Darmstadt (DE); MARX, Stefan, 64380 Roßdorf (DE); TRETZEL, Joachim, 64342 Seeheim-Jugenheim (DE)
(74) Vertreter: dompatent von Kreisler Selting Werner - Partnerschaft von Patent- und Rechtsanwälten mbB
(86) Internationale Anmeldenummer: PCT/EP2016/069743
(87) Internationale Veröffentlichungsnummer: WO 2017/032722

(56) Entgegenhaltungen:
- EP-A1- 1 157 701
- WO-A1-2005/075614
- WO-A1-2015/173705
- ES-A1- 2 233 208
- RAFFAELLA BRIANTE ET AL: "Production of highly purified hydroxytyrosol from Olea europaea leaf extract biotransformed by hyperthermophilic [beta]-glycosidase", JOURNAL OF BIOTECHNOLOGY, Bd. 111, Nr. 1, 1. Juli 2004 (2004-07-01), Seiten 67-77, XP055243787, NL ISSN: 0168-1656, DOI: 10.1016/j.jbiotec.2004.03.011
- HEDYA JEMAI ET AL: "Antidiabetic and Antioxidant Effects of Hydroxytyrosol and Oleuropein from Olive Leaves in Alloxan-Diabetic Rats", JOURNAL OF AGRICULTURAL AND FOOD CHEMISTRY, Bd. 57, Nr. 19, 14. Oktober 2009 (2009-10-14), Seiten 8798-8804, XP055243561, US ISSN: 0021-8561, DOI: 10.1021/jf901280r
- Dimitrious Boskou: "Olive and Olive oil Bioactive Constituents", 15. August 2015 (2015-08-15), Elsevier, Urbana, XP002753351, ISBN: 978-1-630670-41-2 Seite 100,101,336, das ganze Dokument
- Ioannis Nikolaos Therios: 1. Januar 2009 (2009-01-01), CABI, Oxfordshire, XP002753352, ISBN: 978-1-84593-458-3 Seite 29, das ganze Dokument
- SEDEF N EL ET AL: "Olive tree ( Olea europaea ) leaves: potential beneficial effects on human health", NUTRITION REVIEWS, Bd. 67, Nr. 11, 1. November 2009 (2009-11-01), Seiten 632-638, XP055136530, ISSN: 0029-6643, DOI: 10.1111/j.1753-4887.2009.00248.x
- Elaine Watson: "New olive leaf ingredient boasts 25% hydroxytyrosol for cardio health benefits", Nutraingredients-Usa.Com, 21. November 2011 (2011-11-21), Seiten 1-1, XP055243560, Gefunden im Internet: URL:http://www.nutraingredients-usa.com/co ntent/view/print/577267 [gefunden am 2016-01-21]

## Beschreibung

Die Erfindung betrifft einen Olivenblattextrakt, seine Herstellung und seine Verwendung.

Polyphenolen, insbesondere Polyphenolen aus Olivenölen werden gesundheitlich positive Wirkungen zugeschrieben. Der Verzehr von Olivenölen gilt als hilfreich zur Vorbeugung von Gefäßerkrankungen.

In der EU darf für Olivenöl z.B. ein entsprechender "health claim" *"consumption of olive oil polyphenols contributes to the protection of blood lipids from oxidative damage"* verwendet werden.

Die Verwendbarkeit von Olivenöl ist jedoch beschränkt auf gewisse Einsatzgebiete, die auch als mediterrane Diät bezeichnet werden. Es wäre grundsätzlich wünschenswert, die positiven Wirkungen des Olivenöls auch in andere Lebensmittel übertragen zu können.

Aufgabe der vorliegenden Erfindung war es daher, alternative, polyphenolreiche Zusammensetzungen bereit zu stellen.

Gelöst wird die Aufgabe durch einen Olivenblattextrakt enthaltend die folgenden Polyphenolderivate des Olivenöls (Secoiridoidderivate):
I) 3,4-DHPEA-EDA und 4-HPEA-EDA
II) 3,4-DHPEA-EA und 4-HPEA-EA
wobei das Gewichtsverhältnis von I) : II) im Bereich von 0,5:1 bis 50:1 liegt.

Als wässriger Extrakt enthält der Olivenblattextrakt Wasser, beispielsweise in Mengen von mindestens 50 Gew.-% oder mindestens 60 Gew.-% oder mindestens 70 Gew.-%.

Erfindungsgemäß kann überraschenderweise aus Olivenblättern von Oliven bei geeigneter Auswahl der Blätter ein Extrakt erhalten werden, der in seiner Zusammensetzung von Secoiridoidderivaten dem von Olivenöl, und zwar sogar von Olivenöl der extra vergine Qualität (EVOO) entspricht oder nahekommt. Da es sich dabei um einen wässrigen Extrakt handelt, kann er ohne Entmischungserscheinungen beispielsweise wasserhaltigen Lebensmitteln wie Getränken zugesetzt werden.

Grundsätzlich sind Verfahren zur Herstellung von Olivenblattextrakten bekannt.

Briante et al., Journal of Biotechnology, 111, (2004), 67 bis 77 beschreibt ein Verfahren, bei dem aus einen Olivenblattextrakt Hydroxytyrosol gewonnen wird, in dem eine hyperthermophile β-Glycosidase zugesetzt wird. Die zugesetzte β-Glycosidase fördert die Bildung von Hydroxtyrosol. Durch die Verwendung von 50%igem Ethanol als Extraktionsmittel werden endogene Enzyme gehemmt, so dass keine Bildung der erfindungsgemäßen Verbindungen erfolgt.

ES 2 233 208 betrifft ein Verfahren zur Herstellung von Hydroxytyrosol aus Oliven oder Olivenblättern. Es wird beschrieben, dass die Olivenblätter mit einer starken Säure wie Schwefelsäure behandelt werden, um einen pH-Wert im Bereich von 1 oder weniger zu erreichen (siehe Beispiel 1). Dies dient dazu, Strukturen wie Oleuropein zu hydrolysieren. Durch diesen Säurebehandlungsschritt können die Enzyme keine Wirkung entfalten.

WO 2015/173705 beschreibt ein Dekokt von Olivenblättern. Da der Dekokt gekocht wird, sind endogene Enzyme nicht mehr aktiv.

H. Jemai et al., J. Agric. Food Chem. 57 (2009), 8798 bis 8804, beschreiben ein Verfahren zur Bestimmung der antidiabetischen und antioxidativen Effekte von Hydroxytyrosol oder Oleuropein aus Olivenblättern. Der Extrakt wird mit 80%igen Methanol hergestellt. Durch die Verwendung von hochalkoholhaltige im Extraktionsmittel wird die endogene Enzymaktivität gehemmt oder zerstört.

Im Gegensatz zum Stand der Technik soll erfindungsgemäß die endogene Enzymaktivität der Olivenblätter genutzt werden, um Secoiridoidderivate der Gruppen I) und II) zu erhalten.

Bevorzugt liegt das Gewichtsverhältnis der Secoiridoidderivate I) zu den Secoiridoidderivaten II) in den erfindungsgemäßen Extrakten im Bereich von 1:1 bis 15:1. DHPEA und HPEA stehen für (Di-)Hydroxyphenylethylalcohol. EA steht für "elenolic acid". EDA steht für "elenolic decarboxymethyl acid".

### 3,4-DHPEA-EDA hat folgende Struktur

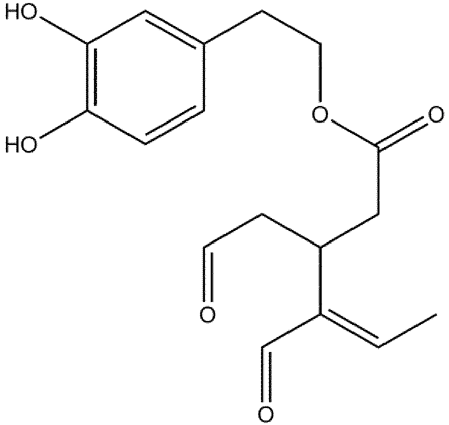

### 4-HPEA-EDA hat folgende Struktur

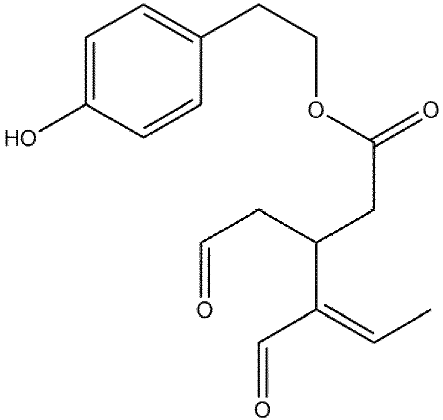

### 3,4-DHPEA-EA ist das Oleuropein-Aglycon und hat folgende Struktur

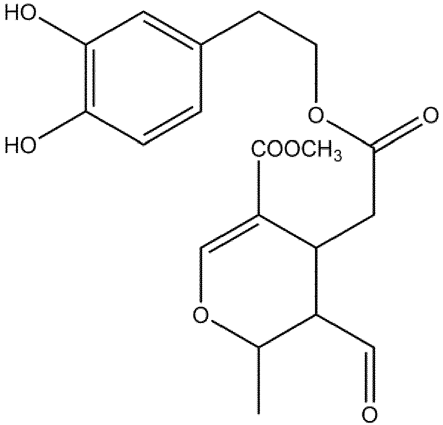

### 4-HPEA-EA ist das Ligstrosid-Aglycon und hat folgende Struktur

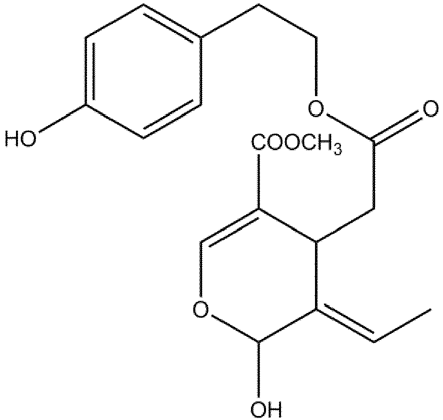

3,4-DHPEA-EA und 4-HPEA-EA kommen sowohl als Halbacetal als auch als Monoaldehyd vor. Erfindungsgemäß werden die Gehalte an 3,4-DHPEA-EDA und 4-HPEA-EDA und 3,4-DHPEA-EA und 4-HPEA-EA gemessen und ihre jeweiligen Gewichtsanteile summiert. Es können in beiden Gruppen einzelne Substanzen auch nicht vorhanden sein, beispielsweise kann der Olivenblattextrakt nur 3,4-DHPEA-EDA und 4-HPEA-EA enthalten.

Bevorzugt liegt der Gehalt an Verbindungen der Gruppe I) und II) im Extrakt bei mindestens 1 mg/ml, bevorzugt bei mindestens 5 oder mindestens 10 oder mindestens 30 mg/ml, oder bei mindestens 1 mg/g bezogen auf den trockenen Extrakt.

Die erfindungsgemäßen Extrakte enthalten typischerweise folgende weitere Secoiridoidderivate:
- Hydroxyphenylethanol-Derivate, insbesondere Hydroxytyrosol und Tyrosol
- Elenolsäurederivate wie Elenolsäure, insbesondere (4E)-4-formly-3-(1-formly-2-methoxy-2-oxoethyl)hex-4-enoic acid und/oder (4E)-4-formyl-3-(2-oxoethyl)hex-4-enoic acid
- Oleuropein und Ligstrosid.

In einer Ausführungsform liegen die Gewichtsverhältnisse entsprechender Verbindungen im Extrakt relativ zur Verbindung der Gruppe II)
- Hydroxytyrosol und Tyrosol: Gruppe II im Verhältnis 0:1 bis 3:1
- Elenolsäure: Gruppe II im Verhältnis 0:1 bis 3:1
- Oleuropein und/oder Ligstrosid: Gruppe II im Verhältnis 0:1 bis 2:1.

Gegenstand der Erfindung ist auch ein Verfahren zur Herstellung der Olivenblattextrakte. Das Verfahren umfasst die Schritte:
a) Auswahl von Olivenblättern, deren Gehalt an Oleuropein plus Ligstrosid im Bereich von 0,1 bis 15 Gew-% bezogen auf das Trockengewicht liegt
b) Vermischen der Olivenblätter mit Wasser, um eine Maische zu erhalten
c) Inkubation der Maische
d) Abtrennen der wässrigen Phase, um einen Olivenblattextrakt zu erhalten.

Durch die Auswahl der Olivenblätter im Schritt a) werden Olivenblätter erhalten, die eine besondere Qualität aufweisen.

In einer Ausführungsform der Erfindung werden die Olivenblätter zusätzlich auf ihren Gehalt an endogenen Enzymen, die zur Bildung von Substanzen der Gruppe I) aus Oleuropein und Ligstrosid führen, ausgewählt.

Die Bestimmungsverfahren für die Gehalte an Oleuropein und Ligstrosid sowie für die Messung der endogenen Enzymaktivität sind in den Beispielen beschrieben.

Die Olivenblätter, die die gewünschten Qualitätsmerkmale aufweisen, werden im nächsten Schritt mit Wasser angemaischt. Hierzu werden die Blätter vor oder während des Maischens mechanisch zerkleinert. Es entsteht dabei ein Brei. Das Gewichtsverhältnis von Olivenblättern zu Wasser liegt bevorzugt im Bereich von 1:3 bis 1:20.

Anschließend wird die Maische inkubiert. Dies erfolgt bevorzugt bei einer Temperatur von etwa 4 bis 80°C für 1 bis 24h. Bevorzugt liegt die Temperatur während der Inkubation im Bereich von 20°C bis 60°C.

Als nächstes erfolgt die Abtrennung der wässrigen Phase von der Maische. Die wässrige Phase ist die Produktphase, die die gewünschten Secoiridoidderivate enthält. Geeignete Mittel zur Abtrennung sind beispielsweise Dekanter, eine Bandpresse, eine Siebpresse oder Bucherpresse. Weitere Verfahren sind dem Fachmann bekannt.

In Abhängigkeit von dem verwendeten Trennverfahren kann sich eine weitere Abtrennung, beispielsweise über einen Schichtenfilter, anbieten.

Nach Abtrennung der wässrigen Phase kann gewünschtenfalls der pH-Wert des Extraktes eingestellt werden. Soweit die Produkte später für Lebensmittel verwendet werden sollen, sind beispielsweise Genusssäuren besonders geeignet.

In einer bevorzugten Ausführungsform wird der Extrakt zumindest teilweise eingeengt, beispielsweise durch Vakuumverdampfer oder Umlaufverdampfer. In vielen Fällen ist es wünschenswert, die Produkte nicht in einen Trockenextrakt zu überführen, sondern als Konzentrat bereitzustellen. Es kann dann in diesem Zustand auch pasteurisiert werden, um die Lagerfähigkeit zu erhöhen.

Gegenstand der Erfindung ist auch ein Lebensmittel, das den erfindungsgemäßen Olivenblattextrakt enthält; geeignete Lebensmittel sind insbesondere Getränke, bevorzugte Getränke sind Teegetränke, Aqua-Plus-Getränke, Fruchtsaftgetränke, Sportgetränke, Energiegetränke oder Funktionsgetränke.

Gegenstand der Erfindung ist weiterhin ein Grundstoff oder ein Konzentrat enthaltend den erfindungsgemäßen Olivenblattextrakt. Solche Grundstoffe oder Konzentrate werden dazu verwendet, unter Verdünnung und gegebenenfalls Zusatz weiterer Zutaten wie Süßungsmitteln oder Genusssäuren ein verzehrfertiges Produkt zu erhalten. Solche Konzentrate und Grundstoffe können sowohl beim Endverbraucher oder beispielsweise beim Abfüller zu verzehrfertigen Produkten ausgemischt werden.

In einer besonderen Ausführungsform der Erfindung wird der Maische ein Enzympräparat zugesetzt. Solche Enzympräparate weisen bevorzugt glycosidische, cellulytische und/oder Esteraseaktivität auf.

Beispielhaft können folgende Enzympräparate verwendet werden, die kommerziell erhältlich sind:
- Lallzyme, erhältlich von der Firma Lallemand Wien, Austria
- Beerzym in den Ausführungsformen Combi, Penta, BG-Super oder Fructozym NAR, erhältlich von der Firma Erbslöh, Geisenheim
- die Enzymzubereitungen Brewers compass, Filtrase, Rapidase, erhältlich von der Firma DSM, Düsseldorf
- Cellulase, erhältlich von der Firma ASA, Wolfenbüttel
- Rohalase, Rohament, erhältlich von der Firma ABEnzymes, Darmstadt sowie
- Panzym Arome G /Extrakt G erhältlich von der Firma Begerow/Eaton, Langenlonsheim.

Die Menge an Enzym liegt bei etwa 0,001 bis 0,5 Gew.-% der kommerziell erhältlichen Zubereitung bezogen auf das Gewicht der Blätter. Bei einem hohen Gehalt an endogenen Enzymen kann ein geringer Zusatz verwendet werden. Durch das Verhältnis von endogenen und exogenen Enzymen kann das Verhältnis der Secoiridoidderivate I) und II) gesteuert werden. Soweit die endogenen Enzymaktivitäten ausgesprochen hoch sind, kann die Aktivität der endogenen Enzyme ganz oder teilweise durch einen Erhitzungsschritt, z.B. während des Maischens reduziert werden.

In vielen Fällen wird es sinnvoll sein, nach der Inkubation ebenfalls einen Erhitzungsschritt durchzuführen, um endogene und/oder exogene Enzyme zu inaktivieren.
Figur 1 zeigt ein Chromatogramm des Secoiridoidspektrums des Olivenöls Med, einem Olivenöl der extravergine Qualität (EVOO) aus der Olivensorte Arbequina.
   - 1) Hydroxyphenylethanol-Derivat
   - 2) Elenolsäurederivat
   - 3) Secoiridoid-Derivate - Gruppe I
   - 4) Secoiridoid-Derivate - Gruppe II
Figur 2 zeigt ein Chromatogramm des Secoiridoidspektrums des Olivenöls Casa del AGUA, einem Olivenöl der extravergine Qualität (EVOO) aus der Olivensorte Arbequina.
   - 1) Hydroxyphenylethanol-Derivat
   - 2) Elenolsäurederivat
   - 3) Secoiridoid-Derivate - Gruppe I
   - 4) Secoiridoid-Derivate - Gruppe II
Figur 3 zeigt ein Chromatogramm mit Secoiridoidspektrum zur Bestimmung der endogenen Enzymaktivität von Olivenblättern. Gemessen wurde das Polyphenolspektrum nach 24stündiger Inkubation ausgehend von 5% Oleuropein. Aufgrund der Verminderung des Gehalts an Oleuropein und der Bildung von Substanzen der Gruppe I) kann auf die Enzymaktivität geschlossen werden.
   - 3) Secoiridoid-Derivate - Gruppe I
   - 4) Secoiridoid-Derivate - Gruppe II
Figur 4 zeigt ein Secoiridoidspektrum des erfindungsgemäß erhaltenen Extrakts nach Beispiel 4.
   - 1) Hydroxyphenylethanol-Derivat
   - 2) Elenolsäurederivat
   - 3) Secoiridoid-Derivate - Gruppe I
   - 4) Secoiridoid-Derivate - Gruppe II
   - 5) Oleuropein/Ligstrosid
Figur 5 zeigt ein Secoiridoidspektrum des Vergleichsextraktes gemäß Beispiel 6.
Figur 6 zeigt ein Secoiridoidspektrum eines Vergleichsextraktes gemäß Beispiel 7.

Die Erfindung wird durch die nachfolgenden Beispiele näher erläutert.

### Beispiel 1: Bestimmung des Gehaltes an Oleuropein (OP)/Ligstrosid (LS)

5 g getrocknete Blätter werden mit 50 ml Leitungswasser gemischt und für 1h bei 80°C unter Rühren inkubiert. Im Anschluss werden die Blätter abgetrennt. Der Überstand wird filtriert und die Konzentration der Secoiridoide mittels HPLC bestimmt (siehe Beispiel 3).

### Beispiel 2: Charakterisierung der endogenen Enzymausstattung

5 g getrocknete Blätter werden mit 50 mL Leitungswasser gemischt und für 24h bei 25°C unter Rühren inkubiert. Im Anschluss werden die Blätter abgetrennt. Der Überstand wird filtriert und die Enzymaktivität im Überstand durch Bestimmung der Produktkonzentration ausgehend von der Konzentration an Oleuropein ermittelt. Für das Verfahren geeignete Blätter besitzen eine endogene Enzymaktivität, die die Bildung von mindesten 0,01 g Secoiridoidderivate der Gruppen I) und II) pro h ausgehend von 5% Oleuropein katalysiert. Der Oleuropein-Gehalt wurde entsprechend durch Zugabe von Oleuropein eingestellt. Substrat und Secoiridoidderivate der Gruppe I) und II) werden mittel RP-HPLC-DAD quantifiziert und der zeitabhängige Umsatz ermittelt; siehe Beispiel 3.

### Beispiel 3: HPLC Bestimmung

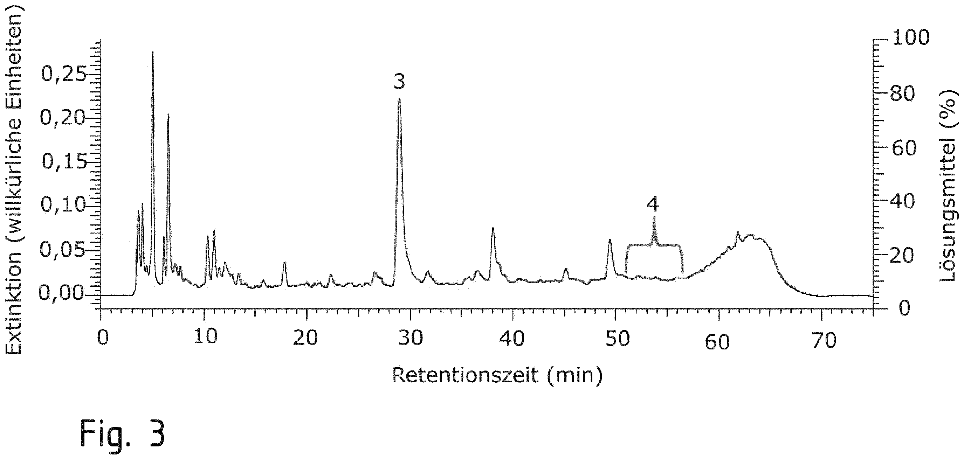

Die Proben werden verdünnt, um eine Endkonzentration von 50% Methanol in Wasser zu erreichen, und zentrifugiert. Der Überstand wird durch einen Spritzenfilter (0,2 µm) in ein HPLC-Gefäß überführt. Secoiridoidderivate werden mittels RP-HPLC-DAD (Agilent System) analysiert. Die Trennung der Secoiridoidderivate erfolgt über eine Reversed Phase C18-Säule (Phenomenex, Luna 5u C18 10A; 250 x 4,6 mm) unter Verwendung eines Laufmittelgradienten aus 0.1% TFA in Wasser (Laufmittel A) und 0.1% TFA in 90% MeOH (Laufmittel B) bei konstant 25°C. 10 µL der Probe wurden über einen Autosampler (Agilent) in das System gegeben und bei 0.5 ml/min mit folgendem Gradienten eluiert: 0 min 30% Laufmittel B, 50 min 50% Laufmittel B, 55 min 60% Laufmittel B, 60 min 70 % Laufmittel B, 65 min 30% Laufmittel B. Die Detektion erfolgte bei 233 nm und 240 nm. Der Gehalt an Ligstrosid und Oleuropein wurde als Peakfläche bei 233 nm Detektion bestimmt. Soweit Derivate von Ligstrosid und Oleuropein bestimmt werden sollte, erfolgte dies durch Detektion bei 240 nm; siehe Figur 3.
Die Analyse von OP/ Hydroxytyrosol (HT)/ Tyrosol (T) erfolgte mit externen Standards erhältlich von der Firma Extrasynthese, Genay Cedex, Frankreich. Weitere Referenzsubstanzen wurden aus Olivenöl in Anlehnung an Montedoro et al. 1993, Impellizzeri and Lin (2006), Carrasco-Pancorbo et al. 2006 gewonnen.

### Beispiel 4: Herstellung des Olivenblattextraktes

9000 L Stadtwasser mit 40 bis 50 °C werden in einen Tank gepumpt und 1000 kg Blätter dazugegeben. Unter schonender Durchmischung erfolgt die Zugabe des Enzyms Beerzym (Erbslöh) mit einer Dosage von 0,2 Gew.-% und weitere Inkubation für 24 h bei 45°C. Zur Reduktion von Oxidationsprozessen wird der Kopfraum regelmäßig mit N₂ begast. Nach Inkubation werden die Blätter mittels eines Dekanters (Gea Westfalia) abgetrennt. Der erhaltene Extrakt wird mit Zitronensäure auf pH 3,5 eingestellt und Trubstoffe mittels Separator entfernt. Der von Trubstoffen befreite Extrakt wird mittels Schichtenfiltration (Erbslöh, J16, 1,5 - 3,0 µm) filtriert und mittels Vakuumverdampfer um den Faktor 10 aufkonzentriert. Ca. 900 kg Konzentrat mit einem Brix-Gehalt von 30° wurden pasteurisiert (95°C, 30 sec.), auf 20°C abgekühlt und abgefüllt.

Das Gewichtsverhältnis der Gruppe I) zu II) liegt bei 2:1.

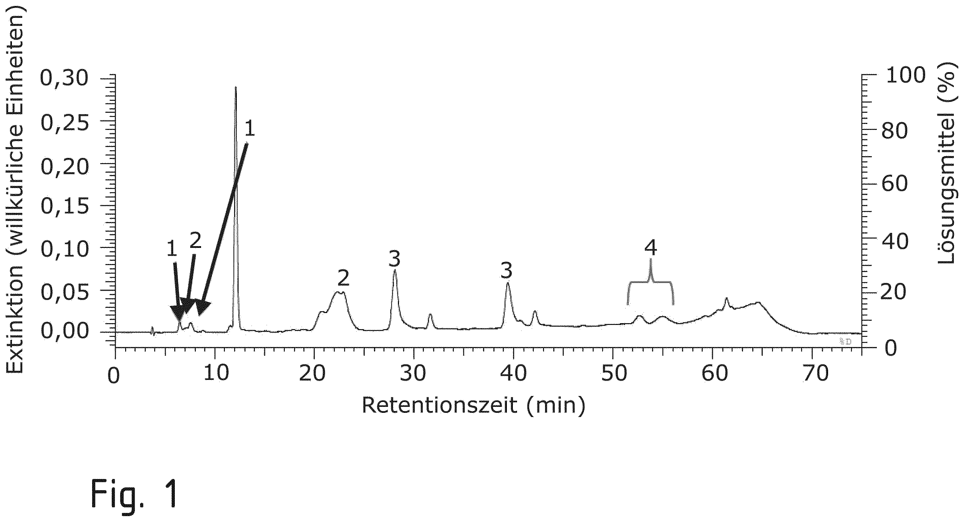

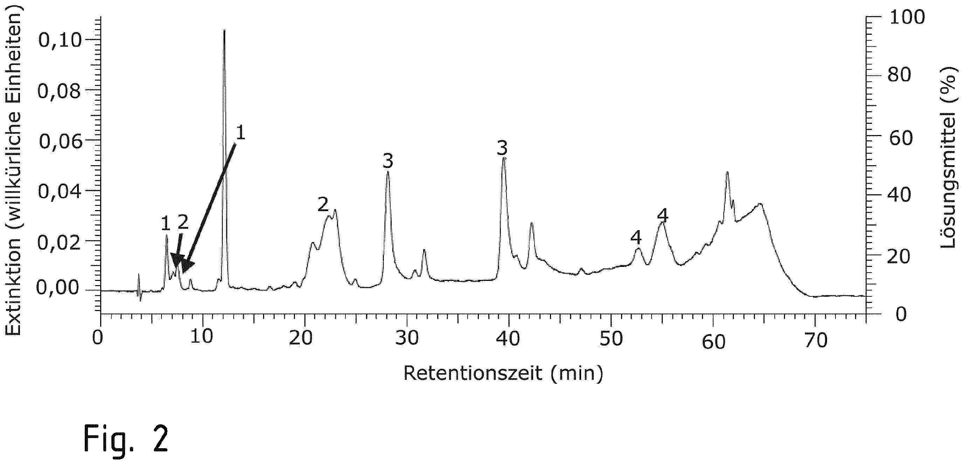

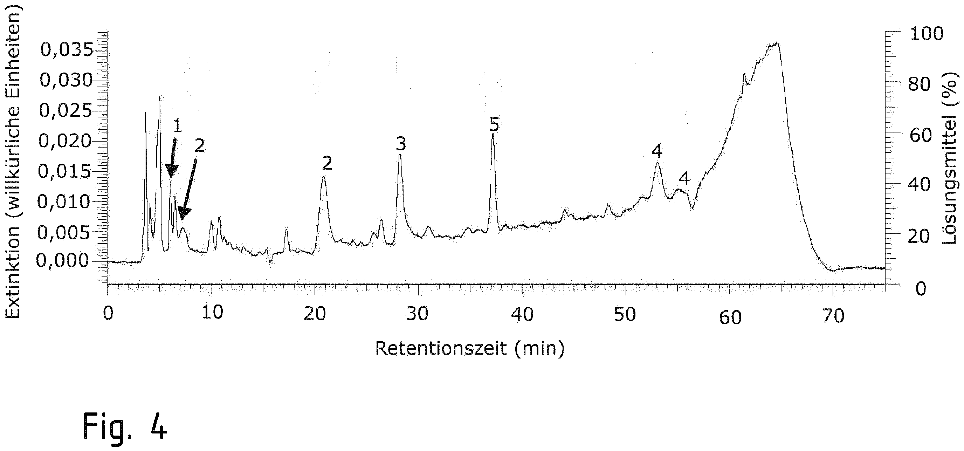

Ein Chromatogramm des erhaltenen Extraktes ist als Figur 4 beigefügt. Im Vergleich zu dem Secoiridoidspektren von zwei verschiedenen Olivenölen (Figur 1, Figur 2) ist zu sehen, dass das Secoiridoidspektrum im Wesentlichen dem von Olivenöl der Qualität EVOO entspricht.

### Beispiel 5: Herstellung eines Getränks

Ein Getränkegrundstoff enthaltend 40 g Agavendicksaft als Süßungskomponente, 0,3 g Säuerungsmittel Zitronensäure, 0,4 g natürliches Zitronenaroma wurde mit 1,17 g des erfindungsgemäßen Polyphenolextraktes nach Beispiel 4 versetzt und im Verhältnis 1:23 mit Wasser vermischt. Es entstand ein wohlschmeckendes Getränk mit hohem Gehalt an gesundheitsförderndem Polyphenol.

### Beispiel 6: Vergleichsextrakt bei hohen Temperaturen

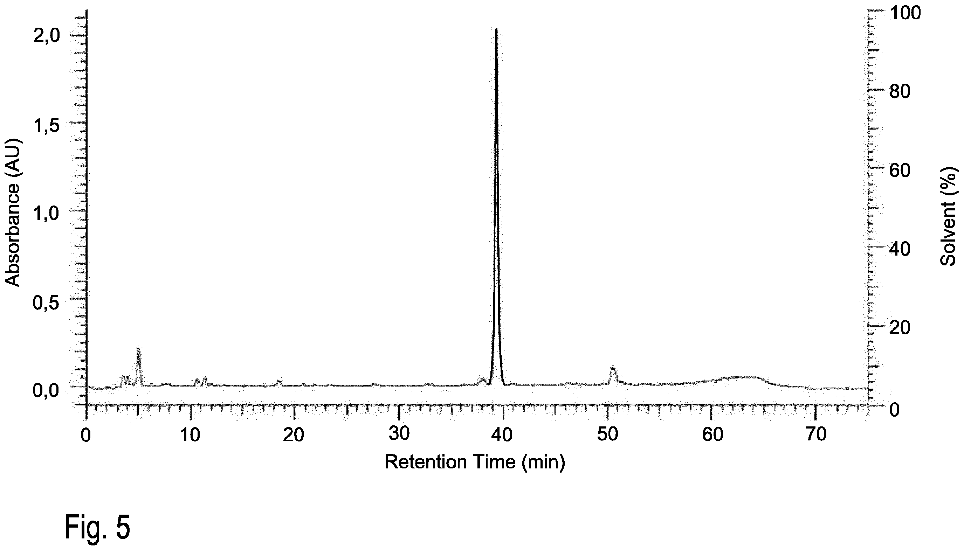

18 kg Wasser werden mit 90°C in einem Gefäß vorgelegt und 2 kg Olivenblätter zugesetzt. Anschließend erfolgt eine Zugabe des Enzyms Beerzym in einer Dosage von 0,2 Gew.-% und eine 1,5 stündige Extraktion bei 90°C. Die weitere Aufarbeitung erfolgt wie im Beispiel 4. Ein entsprechendes Chromatogramm ist als Figur 5 beigefügt. Es werden keine Substanzen I und II erhalten, sondern nur Oleuropein und geringe Mengen Hydroxytyrosol. Durch die hohen Temperaturen werden sowohl die endogenen als auch die exogenen Enzyme inaktiviert und die chemische Hydrolyse von Oleuropein gefördert.

### Beispiel 7: Vergleichsextrakt mit Ethanol

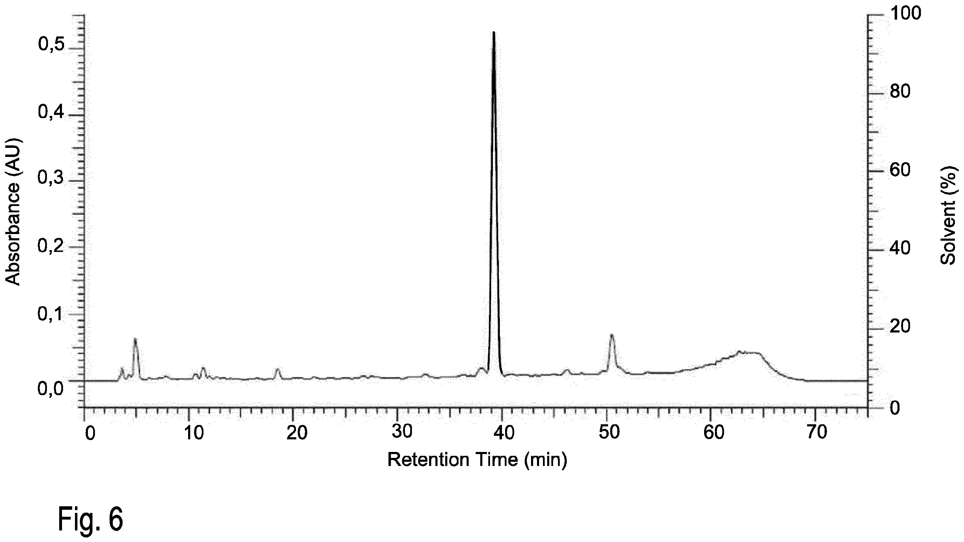

12,5 kg Ethanol 96%ig wurden bei 22°C in einem Behälter vorgelegt und 2,38 kg Olivenblätter hinzugefügt. Es erfolgte die Zugabe des Enzymes Beerzym in einer Dosage von 0,2 Gew.-% und eine Extraktion für 1,5 Stunden. Figur 6 zeigt ein Chromatogramm des erhaltenen Extraktes. Der Extrakt enthält nahezu nur Oleuropein und geringe Mengen an Hydroxytyrosol. Das alkoholische Extraktionsmittel inhibiert endogene und exogene Enzyme.

### Literaturverzeichnis:

- Montedoro G., Servili M., Baldioli M., Selvaggini R., Miniati E., Macchioni E., J. Agric. Food Chem., 1993, 41 (11), pp 2228-2234. "Simple and hydrolyzable compounds in virgin olive oil. 3. Spectroscopic characterizations of the secoiridoid derivatives".
- Impellizzeri J, Lin J. J Agric Food Chem. 2006 May 3;54(9):3204-8. "A simple high-performance liquid chromatography method for the determination of throat-burning oleocanthal with probated antiinflammatory activity in extra virgin olive oils".
- Carrasco-Pancorbo A, Cerretani L, Bendini A, Segura-Carretero A, Del Carlo M, Gallina-Toschi T, Lercker G, Compagnone D, Fernández-Gutiérrez A.J Agric Food Chem. 2005 Nov 16;53(23):8918-25. "Evaluation of the antioxidant capacity of individual phenolic compounds in virgin olive oil".

Alle zitierten Dokumente sind im vollen Umfang mit in die Offenbarung einbezogen, soweit diese Offenbarung nicht im Widerspruch zu der Lehre der Erfindung steht.

## Patentansprüche

1. Olivenblattextrakt enthaltend die folgenden Secoiridoidderivate
I) 3,4-DHPEA-EDA und 4-HPEA-EDA
II) 3,4-DHPEA-EA und 4-HPEA-EA
wobei das Gewichtsverhältnis von I) : II) im Bereich von 0,5:1 bis 50:1. liegt.

2. Olivenblattextrakt nach Anspruch 1 wobei das Gewichtsverhältnis von I) : II) im Bereich von 1:1 bis 15:1 liegt.

3. Olivenblattextrakt nach Anspruch 1 oder 2 wobei zusätzliche Secoiridoid-Derivate, ausgewählt aus der Gruppe
- Hydroxyphenylethanol-Derivate, insbesondere Hydroxytyrosol und Tyrosol
- Elenolsäurederivate wie Elenolsäure, insbesondere (4E)-4-formly-3-(1-formly-2-methoxy-2-oxoethyl)hex-4-enoic acid und/oder (4E)-4-formyl-3-(2-oxoethyl)hex-4-enoic acid
- Oleuropein und/oder Ligstrosid
enthalten sind.

4. Olivenblattextrakt nach Anspruch 3, wobei Secoiridoid-Derivate in folgenden Gewichtsverhältnissen zur Gruppe II vorhanden sind:
- Hydroxytyrosol und Tyrosol: Gruppe II im Verhältnis 0:1 bis 3:1
- Elenolsäure: Gruppe II im Verhältnis 0:1 bis 3:1
- Oleuropein und Ligstrosid: Gruppe II im Verhältnis 0:1 bis 2:1.

5. Verfahren zur Herstellung eines Olivenblattextraktes nach einem der Ansprüche 1 bis 4 umfassend die Schritte:
a) Auswahl von Olivenblättern, deren Gehalt an Oleuropein plus Ligstrosid im Bereich von 0,1 bis 15 Gew-% bezogen auf das Trockengewicht liegt
b) Vermischen der Olivenblätter mit Wasser, um eine Maische zu erhalten
c) Inkubation der Maische bei einer Temperatur von 4 bis 80°C für 1 bis 24 h
d) Abtrennen der wässrigen Phase, um einen Olivenblattextrakt zu erhalten.

6. Verfahren nach Anspruch 5, wobei zusätzlich die Auswahl von Olivenblättern nach dem Gehalt an endogenen Enzymen, die zur Bildung von Substanzen der Gruppe I) aus Oleuropein und Ligstrosid führen, erfolgt.

7. Verfahren zur Herstellung eines Olivenblattextraktes nach Anspruch 5 oder 6 umfassend den Schritt
- Zugabe von Enzympräparaten zur Maische.

8. Verfahren nach Anspruch 7, wobei die Enzympräparate glycosidische, cellulytische und/oder Esteraseaktivität aufweisen.

9. Verfahren zur Herstellung eines Olivenblattextraktes nach einem der Ansprüche 5 bis 8 umfassend den Schritt
- Inaktivierung von Enzymen in der wässrigen Phase durch Hitze und/oder pH-Wert-Reduzierung

10. Verfahren nach einem der Ansprüche 5 bis 9, wobei die Inkubation der Maische bei 4 bis 80°C für 1 bis 24h erfolgt.

11. Verfahren nach einem der Ansprüche 5 bis 10, wobei das Abtrennen der wässrigen Phase über einen Dekanter, eine Bandpresse oder eine Sieb- oder Bucherpresse erfolgt.

12. Verfahren nach einem der Ansprüche 5 bis 10, wobei die wässrige Phase eingeengt wird, ggf. bis zur Trockene, wobei ein Pulver erhalten wird.

13. Lebensmittel enthaltend den Olivenblattextrakt nach einem der Ansprüch 1 bis 4.

14. Lebensmittel nach Anspruch 13, wobei das Lebensmittel ein Getränk ist, insbesondere wobei das Getränk ausgewählt ist aus Teegetränken, Aqua-Plus-Getränken, Fruchtsaftgetränken, Sportgetränken, Energiegetränken, Funktionsgetränken.

15. Grundstoff oder Konzentrat enthaltend einen Olivenblattextrakt gemäß einem der Ansprüche 1 bis 4.

## Claims

1. An olive leaf extract, comprising the following seco-iridoid derivatives:
I) 3,4-DHPEA-EDA and 4-HPEA-EDA
II) 3,4-DHPEA-EA and 4-HPEA-EA;
wherein the weight ratio of I) : II) is within a range of from 0.5:1 to 50:1.

2. The olive leaf extract according to claim 1, wherein the weight ratio of I) : II) is within a range of from 1:1 to 15:1.

3. The olive leaf extract according to claim 1 or 2, wherein additional secoiridoid derivatives selected from the group of
- hydroxyphenylethanol derivatives, especially hydroxytyrosol and tyrosol
- elenolic acid derivatives, such as elenolic acid, especially (4E)-4-formyl-3-(1-formyl-2-methoxy-2-oxoethyl)hex-4-enoic acid and/or (4E)-4-formyl-3-(2-oxoethyl)hex-4-enoic acid
- oleuropein and/or ligstroside
are contained therein.

4. The olive leaf extract according to claim 3, wherein seco-iridoid derivatives are present in the following weight ratios to group II:
- hydroxytyrosol and tyrosol: group II at a ratio of 0:1 to 3:1
- elenolic acid: group II at a ratio of 0:1 to 3:1
- oleuropein and ligstroside: group II at a ratio of 0:1 to 2:1.

5. A process for preparing an olive leaf extract according to any of claims 1 to 4, comprising the steps of:
a) selecting olive leaves in which the content of oleuropein plus ligstroside is within a range of from 0.1 to 15% by weight, based on the dry weight;
b) mixing the olive leaves with water to obtain a mash;
c) incubating the mash at a temperature of 4 to 80 °C for 1 to 24 hours;
d) separating off the aqueous phase to obtain an olive leaf extract.

6. The process according to claim 5, wherein the selection of olive leaves is additionally effected by the content of endogenous enzymes that lead to the formation of substances of group I) from oleuropein and ligstroside.

7. The process for preparing an olive leaf extract according to claim 5 or 6, comprising the step of
- adding enzyme preparations to said mash.

8. The process according to claim 7, wherein said enzyme preparations have glycosidic, cellulytic and/or esterase activities.

9. The process for preparing an olive leaf extract according to any of claims 5 to 8, comprising the step of
- inactivating enzymes in the aqueous phase by heat and/or pH reduction.

10. The process according to any of claims 5 to 9, wherein said incubation of the mash is effected at 4 to 80 °C for 1 to 24 hours.

11. The process according to any of claims 5 to 10, wherein said separating off of the aqueous phase is effected using a decanter, a belt press, or a sieve press or Bucher press.

12. The process according to any of claims 5 to 10, wherein the aqueous phase is concentrated, optionally to dryness, to obtain a powder.

13. A foodstuff comprising said olive leaf extract according to any of claims 1 to 4.

14. The foodstuff according to claim 13, wherein said foodstuff is a beverage, especially wherein said beverage is selected from tea beverages, aqua-plus beverages, fruit juice beverages, sports drinks, energy drinks, functional drinks.

15. A base or concentrate comprising an olive leaf extract according to any of claims 1 to 4.

## Revendications

1. Extrait de feuille d'olivier comprenant les dérivés séco-iridoïdes suivants :
I) 3,4-DHPEA-EDA et 4-HPEA-EDA
II) 3,4-DHPEA-EA et 4-HPEA-EA
dans lequel le rapport en poids I):II) est compris dans la gamme allant de 0,5:1 à 50:1.

2. Extrait de feuille d'olivier selon la revendication 1, dans lequel le rapport en poids I):II) est compris dans la gamme allant de 1:1 à 15:1.

3. Extrait de feuille d'olivier selon la revendication 1 ou 2, dans lequel sont compris des dérivés séco-iridoïdes supplémentaires, sélectionnés parmi le groupe
- des dérivés hydroxyphényléthanol, en particulier l'hydroxytyrosol et le tyrosol
- des dérivés de l'acide élénolique tels que l'acide élénolique, en particulier l'acide (4E)-4-formyl-3-(1-formyl-2-méthoxy-2-oxoéthyl)hex-4-ènoique et/ou l'acide (4E)-4-formyl-3-(2-oxoéthyl)hex-4-ènoïque.
- de l'oleuropéine et/ou du ligstroside.

4. Extrait de feuille d'olivier selon la revendication 3, dans lequel les dérivés séco-iridoïdes sont présents dans les rapports en poids suivants par rapport au groupe II :
- hydroxytyrosol et tyrosol:groupe II dans un rapport allant de 0:1 à 3:1
- acide élénolique: groupe II dans un rapport allant de 0:1 à 3:1
- oleuropéine et ligstroside:groupe II dans un rapport allant de 0:1 à 2:1.

5. Procédé de production d'un extrait de feuille d'olivier selon l'une des revendications 1 à 4, comprenant les étapes suivantes :
a) sélection de feuilles d'olivier, dont la teneur en oleuropéine plus ligstroside est comprise dans la gamme allant de 0,1 à 15 % en poids (m/m) par rapport à la masse sèche
b) mélange des feuilles d'olivier avec de l'eau afin d'obtenir un moût
c) incubation du moût à une température allant de 4 à 80 °C pendant 1 à 24 h
d) séparation de la phase aqueuse, afin d'obtenir un extrait de feuille d'olivier.

6. Procédé selon la revendication 5, dans lequel, en outre, la sélection des feuilles d'olivier s'effectue selon la teneur en enzymes endogènes menant à la formation de substances du groupe I) à partir d'oleuropéine et de ligstroside.

7. Procédé de production d'un extrait de feuille d'olivier selon la revendication 5 ou 6, comprenant l'étape
- ajout de préparations enzymatiques au moût.

8. Procédé selon la revendication 5, dans lequel les préparations enzymatiques possèdent une activité glycosidique, cellulolytique et/ou d'estérase.

9. Procédé de production d'un extrait de feuille d'olivier selon l'une des revendications 5 à 8, comprenant l'étape
- inactivation des enzymes dans la phase aqueuse par chauffage et/ou par réduction du pH.

10. Procédé selon l'une des revendications 5 à 9, dans lequel l'incubation du moût s'effectue entre 4 et 80 °C pendant 1 à 24 h.

11. Procédé selon l'une des revendications 5 à 10, dans lequel la séparation de la phase aqueuse s'effectue par le biais d'une presse à bandes ou d'une presse à tamis.

12. Procédé selon l'une des revendications 5 à 10, dans lequel la phase aqueuse est réduite, éventuellement jusqu'à ce qu'elle soit sèche, afin d'obtenir une poudre.

13. Aliment comprenant l'extrait de feuille d'olivier selon l'une des revendications 1 à 4.

14. Aliment selon la revendication 13, dans lequel l'aliment est une boisson, en particulier dans lequel la boisson est sélectionnée parmi les boissons à base de thé, les boissons Aqua-Plus, les boissons à base de jus de fruits, les boissons de sport, les boissons énergisantes, les boissons fonctionnelles.

15. Matière première ou concentré comprenant un extrait de feuille d'olivier selon l'une des revendications 1 à 4.
